(19) Europäisches Patentamt
European Patent Office
Office européen des brevets

(11) Veröffentlichungsnummer: **0 244 786 B1**

(12) # EUROPÄISCHE PATENTSCHRIFT

(45) Veröffentlichungstag der Patentschrift: **06.03.91**

(51) Int. Cl.5: **C07C 239/20**, C07D 333/04, C07D 307/12, C07D 271/10, C07D 317/18, C07D 213/30

(21) Anmeldenummer: **87106351.7**

(22) Anmeldetag: **02.05.87**

Die Akte enthält technische Angaben, die nach dem Eingang der Anmeldung eingereicht wurden und die nicht in dieser Patentschrift enthalten sind.

(54) **Verfahren zur Herstellung von O-substituierten Hydroxylaminen.**

(30) Priorität: **07.05.86 DE 3615473**

(43) Veröffentlichungstag der Anmeldung: **11.11.87 Patentblatt 87/46**

(45) Bekanntmachung des Hinweises auf die Patenterteilung: **06.03.91 Patentblatt 91/10**

(84) Benannte Vertragsstaaten: **AT BE CH DE FR GB IT LI NL**

(56) Entgegenhaltungen:
**AT-B- 316 511**

**CHEMICAL ABSTRACTS, Band 98, Nr. 21, 23. Mai 83, Columbus, Ohio, US; T.J. DELLA "An application of the Mannich reaction using hydroxylamine and some of its derivatives. 6-Substituted-2,4-diamino-5,6,7,8-tetrahydro-pyrimido(4,5-d)pyrimidines" Seite 638, Zusammenfassung-Nr. 198148u**

(73) Patentinhaber: **BASF Aktiengesellschaft**
**Carl-Bosch-Strasse 38**
**W-6700 Ludwigshafen(DE)**

(72) Erfinder: **Wild, Jochen, Dr.**
**An der Marlach 7**
**W-6705 Deidesheim(DE)**
Erfinder: **Goetz, Norbert, Dr.**
**Schoefferstrasse 25**
**W-6520 Worms 1(DE)**
Erfinder: **Will, Wolfgang, Dr.**
**Im Buegen 12**
**W-6719 Kirchheim(DE)**
Erfinder: **Kohler, Rolf-Dieter, Dr.**
**Amselweg 3**
**W-6803 Edingen-Neckarhausen(DE)**
Erfinder: **Plath, Peter, Dr.**
**Hans-Balcke-Strasse 13**
**W-6710 Frankenthal(DE)**

**EP 0 244 786 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung Herstellung von O-substituierten Hydroxylaminen der allgemeinen Formel I,

$$H_2N\text{-}O\text{-}CH_2\text{-}R \qquad (I)$$

in der R Wasserstoff oder einen organischen Rest bedeutet, und deren anorganischen und organischen Salzen durch Umsetzung eines cyclischen Imidethers I

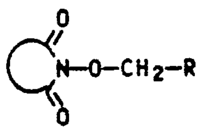

(II)

I einer 1,4- oder 1,5-Dicarbonsäure, der eine Ethergruppierung -O-$CH_2$-R enthält, mit einer basischen Verbindung, dadurch gekennzeichnet, daß man als basische Verbindung einen primären aliphatischen Aminoalkohol III,

$$H_2N\text{-}A\text{-}OH \qquad (III)$$

in der -A- einen zweiwertigen aliphatischen Rest bedeutet, verwendet und die Verbindungen I danach gegebenenfalls in ihre Salze überführt.

Außerdem betrifft die Erfindung neue O-substituierte Hydroxylamine der allgemeinen Formel IV

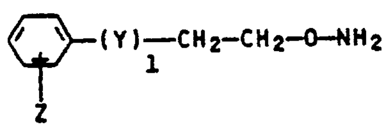

(IV)

in der die Substituenten folgende Bedeutung haben:

Y Sauerstoff oder Schwefel,
Z $C_1$-$C_8$-Alkyl, Halogen oder $CF_3$,
l 0 oder 1

und deren anorganische und organische Salze, die Zwischenprodukte für Pflanzenschutzmittel sind.

Es ist allgemein bekannt, daß die Substitution eines Hydroxylamins zur Reaktion am Aminstickstoff führt. Um die Substitution an der Hydroxygruppe zu erhalten, muß die Aminfunktion im allgemeinen blockiert werden. Die gesamte Synthese gliedert sich daher in drei Schritte:

- Schutz des Hydroxylamins am Stickstoff
- Substitution am freien Sauerstoff
- Abspaltung der Schutzgruppe.

Aus Kostengründen kommen für technische Synthesen vor allem vier N-blockierte Hydroxylaminderivate in Betracht:

- cyclische N-Hydroxyimide, vor allem N-Hydroxyphthalimid,
- Ketonoxime, vor allem Acetonoxim
- Hydroxyurethane
- Hydroxamsäuren und deren Derivate, wie z.B. Esteroxime.

Die Substitution dieser Hydroxylaminderivate am Sauerstoff bereitet in der Regel keine Schwierigkeiten. Wesentlich problematischer gestaltet sich hingegen die Abspaltung der Schutzgruppen und die Abtrennung und Reingewinnung der substituierten Hydroxylamine.

Es ist aus Houben/Weyl, Bd. 10/1, Seite 1181ff bekannt, daß alle vier O-Alkylderivate durch Hydrolyse in wäßriger Mineralsäure, z.T. auch in wäßriger Lauge gespalten werden können. Unter diesen Reaktionsbedingungen laufen jedoch Nebenreaktionen ab, so daß diese Spaltmethode nur begrenzt anwendbar ist zur

Herstellung einfacher und sehr stabiler O-substituierter Hydroxylamine.

Aus Houben-Weyl, Bd. 10/1, Seite 1181ff ist ebenfalls bekannt, daß Phthalimidether wesentlich schonender durch Nucleophile, insbesondere Hydrazine und Amine, in wasserfreiem Medium gespalten werden können.

Aus den DE-PS 22 06 890 und DE-PS 22 41 035 ist die Spaltung mit primären Aminen bekannt, jedoch wenig verbreitet, da die Abtrennung des Produktes von überschüssigem Amin und insbesondere vom N-Alkylphthalimid große Probleme bereitet.

Die Hydrazinolyse von Phthalimidethern zur Herstellung von O-substituierten Hydroxylaminen ist eine oft angewandte Labormethode. Neben dem ausfallenden Phthalsäurehydrazid entsteht jedoch auch das basische N-Aminophthalimid, dessen Abtrennung vom ebenfalls basischen Endprodukt sehr problematisch ist.

Große Schwierigkeiten erwachsen auch bei der Durchführung dieser Reaktion in technischem Maßstab. Das entstehende schwerlösliche Phthalsäurehydrazid fällt als voluminöser Niederschlag aus, so daß zur Handhabung große Mengen an Lösungsmittel und große Reaktionskessel benötigt werden. Die akute Toxizität und der hohe Preis von Hydrazin stehen einer wirtschaftlichen Anwendung ebenfalls hinderlich im Wege.

Ferner ist aus JP-AS-82/48059 bekannt, daß Alkyl, Alkenyl- und Alkinylphthalimidether mit wäßriger Hydroxylaminsulfatlösung gespalten werden können. Die destillative Abtrennung der O-substituierten Hydroxylamine von Hydroxyphthalimid liefert nur wäßrige Lösungen der O-substituierten Hydroxylamine, so daß dieses Verfahren zur Herstellung isolierter (wasserfreier) O-Alkylhydroxylamine oder deren Salze nicht angewendet werden kann.

Zur Verwendung der O-substituierten Hydroxylamine als Zwischenprodukte ist es jedoch wünschenswert, diese in isolierter (wasserfreier) Form oder auch in organischen Lösungsmitteln gegebenenfalls im wasserfreien Medium weiter umzusetzen.

Durch die bei diesem Verfahren notwendige destillative Reinigung können nach dieser Methode nur destillierbare und somit stabile O-substituierte Hydroxylamine von geringem Molekulargewicht erhalten werden.

Der Erfindung lag daher die Aufgabe zugrunde, ein Verfahren zu entwickeln, das die zuvor genannten Nachteile nicht mehr aufweist.

Demgemäß wurde ein Verfahren zur Herstellung von O-substituierten Hydroxylaminen der allgemeinen Formel I und deren anorganischen und organischen Salzen durch Umsetzung eines cyclischen Imidethers II einer 1,4- oder 1,5-Dicarbonsäure, der eine Ethergruppierung $-O-CH_2-R$ enthält, mit einer basischen Verbindung gefunden, welches dadurch gekennzeichnet ist, daß man als Base einen primären aliphatischen Aminoalkohol III verwendet und die Verbindungen I danach gegebenenfalls in ihre Salze überführt.

Außerdem wurde gefunden, daß wasserunlösliche Verbindungen I vorteilhaft unter Verwendung eines Überschusses des Aminoalkohols III über den Imidether II hergestellt und durch wäßrige Extraktion die wasserlöslichen Anteile abgetrennt werden.

Ferner wurden neue O-substituierte Hydroxylamine der Formel IV gefunden.

Die zur Herstellung der Verbindungen II benötigten cyclischen N-Hydroxyimide der zugrundeliegenden 1,4- oder 1,5-Dicarbonsäuren sind entweder bekannt oder können nach bekannten Methoden aus den cyclischen Anhydriden durch Umsetzung mit Hydroxylamin hergestellt werden. Geeignete cyclische N-Hydroxyimide sind beispielsweise:

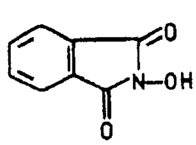

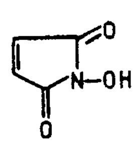

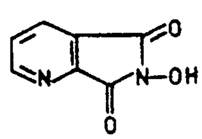

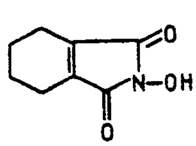

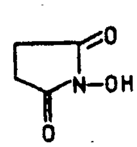

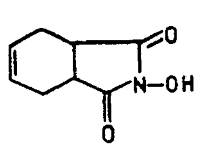

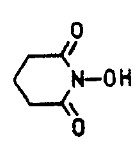

Die cyclischen N-Hydroxyimide können mit Halogeniden oder Sulfonsäureestern $R\text{-}CH_2\text{-}X$ in Gegenwart eines säurebindenden Mittels in die entsprechenden Imidether II überführt werden:

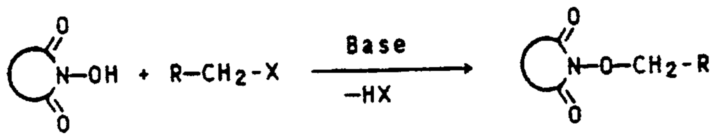


X = z.B. Cl, Br, Mesyl,
Benzolsulfonyl,
Tosyl.

Nach den bisherigen Beobachtungen ist das gute Gelingen des Verfahrens von der Natur der Reste R nicht erkennbar abhängig, sofern diese Reste keine reaktiven Gruppen wie Oxo- oder Säurehalogenidgruppen tragen.

Der Rest R bedeutet beispielsweise

- Wasserstoff,
- eine Alkylgruppe wie Methyl, Ethyl, n-Propyl, iso-Propyl, n-Butyl, iso-Butyl, tert.-Butyl, Hexyl, Octyl, Decyl, Dodecyl, Tridecyl,
- eine Alkenylgruppe wie Ethenyl, 1-Propenyl, 2-Propenyl, Butenyl, Hexenyl,
- eine Alkinylgruppe wie Ethinyl, Propargyl,
- eine Alkoxyalkylgruppe wie Methoxymethyl, Methoxyethyl, Methoxypropyl, Ethoxymethyl, Ethoxyethyl, Ethoxybutyl,
- eine Halogenalkylgruppe wie Trifluormethyl, Fluormethyl, 1-Fluorethyl, Chlorethyl,
- eine Halogenalkenylgruppe wie Chlorethenyl, Fluorethenyl, Bromethenyl,
- eine Cycloalkylgruppe wie Cyclopropyl, Cyclopentyl, Cyclohexyl, Cyclooctyl,
- eine Halogencycloalkylgruppe, wie 2,2-Dichlorcyclopropyl,
- eine Arylgruppe wie Phenyl, Naphthyl,
- eine substituierte Phenylgruppe wie p-Fluorphenyl, p-Tolyl, p-Nitrophenyl, p-Cyanophenyl, m-Chlorphenyl, p-Methoxyphenyl-, m-Trifluormethylphenyl, p-Chlorphenyl, 3,4-Dichlorphenyl, 2,6-Dichlorphenyl, -eine Heteroarylgruppe wie 3-Pyridyl, 5-Isopropyl-1,3,4-oxadiazol-2-yl, 5-Chlorthien-2-yl, 5-Methylthien-2-yl,
- die Benzylgruppe,
- eine substituierte Benzylgruppe wie p-Fluorbenzyl, p-Methylbenzyl, p-Nitrobenzyl, p-Cyanobenzyl, o-Chlorbenzyl, m-Chlorbenzyl, p-Chlorbenzyl,
- eine Phenoxyalkylgruppe wie Phenoxymethyl, Phenoxyethyl, Phenoxybutyl, Phenoxyhexyl,
- eine substituierte Phenoxyalkylgruppe wie o-Fluorphenoxymethyl, m-Trifluormethylphenoxymethyl, o- und m-Chlorphenylmethyl, o-Fluorphenoxyethyl, m-Trifluormethylphenoxyethyl, o- und m-Chlorphenoxyethyl,
- eine Halogencycloalkyl-alkylgruppe wie 2,2-Dichlorcyclopropyl-methyl, 2-Chlorcyclopentyl-ethyl,
- eine Aryloxyalkylgruppe wie 2-Naphthyloxymethyl, 2-Naphthyloxyethyl,
- eine Phenylthioalkylgruppe wie Phenylthiomethyl, Phenylthiobutyl, Phenylthiohexyl,
- eine substituierte Phenylthioalkylgruppe wie m-Chlorphenylthiomethyl, m-Chlorphenylthioethyl,
- eine Dialkoxyalkylgruppe wie Dimethoxyethyl, Diethoxymethyl, Diethoxyethyl, Diethoxybutyl, Diethoxyhexyl,
- eine Phenylalkylgruppe wie Phenylethyl, Phenylpropyl, Phenylbutyl, Phenylhexyl,
- eine substituierte Phenylalkylgruppe wie p- oder m-Chlorphenylethyl,
- eine Carboxylatgruppe wie Methylcarboxylat, Ethylcarboxylat, iso-, tert.-Butylcarboxylat,
- eine Carboxamidgruppe,
- eine N-Alkylcarboxamidgruppe,
- eine N,N-Dialkylcarboxamidgruppe wie N,N-Dimethylcarboxamid,
- eine Nitrilgruppe,
- eine Cyanoalkylgruppe wie Cyanomethyl, 2-Cyanoethyl oder
- einen cyclischen Ether wie 2-Tetrahydrofuryl, 2-Tetrahydrofuryl-methyl, 2-Tetrahydrofurylbutyl.

Unter den primären aliphatischen Aminoalkoholen III eignen sich vor allem solche der Verbindung III,

$$H_2N\text{-}A\text{-}OH \qquad (III)$$

in der -A- für $-(CH_2)_n-$, $-CH_2CH_2(CH_3)CH_2-$ oder $-(CH_2CH_2O)_m-CH_2CH_2-$ steht, wobei m = 1 oder 2 und n

= 2 bis 4 bedeuten; besonders geeignet sind diejenigen Verbindungen III, in denen -A- folgende Bedeutung hat:

- $-CH_2-CH_2-$, $-CH_2-CH_2-CH_2-$, $-CH_2-CH_2-CH_2-CH_2-$,
- $-CH_2-CH(CH_3)CH_2-$,
- $-CH_2CH_2OCH_2CH_2-$ und $-CH_2CH_2O-CH_2CH_2O-CH_2CH_2-$.

Unter den Aminoalkoholen III sind 3-Aminopropan-1-ol, 4-Aminobutan-1-ol, 2-(2-Aminoethoxy)-ethanol sowie vor allem 2-Aminoethanol bevorzugt.

Die erfindungsgemäßen Verbindungen IV sind nach dem zuvor beschriebenen erfindungsgemäßen Verfahren herstellbar. Der Substituent Z, -Y- und der Index 1 haben folgende Bedeutung:

| | | |
|---|---|---|
| Y | - | Sauerstoff und Schwefel, bevorzugt Sauerstoff, |
| Z | - | $C_1$-$C_8$-Alkyl, bevorzugt $C_1$-$C_4$-Alkyl, besonders bevorzugt Methyl und Ethyl, |
| | - | Halogen, bevorzugt Fluor, Chlor und Brom, besonders bevorzugt Chlor, |
| | - | Trifluormethyl, |
| 1 | - | 0 und 1, bevorzugt 0. |

Zur Herstellung der O-substituierten Hydroxylamine durch Umsetzung eines Imidethers II mit Aminoalkohol III sind - abhängig von der Struktur des Restes R - im wesentlichen zwei Reaktionsführungen möglich:

a) Für wasserunlösliche O-substituierte Hydroxylamine I

Dazu trägt man den Imidether II in überschüssigen Aminoalkohol III ein und rührt bei 20 bis 100 °C, vorzugsweise zwischen 40 und 70 °C, bis die Spaltung beendet ist. Die Mischung wird in Wasser gegossen, wobei der überschüssige Aminoalkohol und das Spaltprodukt in Lösung gehen. Das O-substituierte Hydroxylamin I wird entweder abgesaugt oder aus der wäßrigen Phase mit einem geeigneten Lösungsmittel extrahiert.

Zur Extraktion geeignete Lösungsmittel sind chlorierte Kohlenwasserstoffe wie Methylenchlorid, Chloroform, Tetrachlorkohlenstoff, 1,1,1-Trichlorethan, Hexachlorethan, Tetrachlorethylen und Chlorbenzol. Ether wie Diethylether, Methyl-tert.-butylether und Diisopropylether, Carbonsäureester wie Essigsäuremethylester und Essigsäureethylester; besonders bevorzugt werden Methylenchlorid, Essigsäureethylester und Methyl-tert.-butylether.

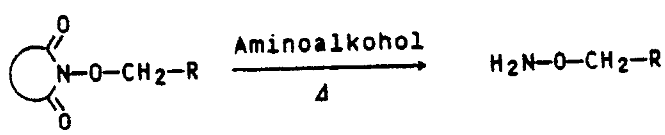


b) Für teilweise und gänzlich wasserlösliche O-substituierte Hydroxylamine I

Sie lassen sich besonders vorteilhaft in Form ihrer Salze isolieren, indem man den Imidether II teilweise oder gänzlich in einem inerten Lösungsmittel löst und äquimolare Mengen oder einen Überschuß zwischen 0,01 bis 30 Mol.% an Aminoalkohol III, vorzugsweise 5 bis 15 Mol.%, zusetzt. Dabei entsteht eine homogene Reaktionsmischung, die bei 20 bis 100 °C, vorzugsweise zwischen 40 und 70 °C, bis zum Ende der Spaltung gerührt wird. Das Spaltprodukt wird abgesaugt und das Filtrat mit einer wasserfreien Säure behandelt, wobei man das Salz des O-substituierten Hydroxylamins in hoher Reinheit und Ausbeute erhält.

Unter den gasförmigen Säuren ist die trockene Chlorwasserstoffsäure besonders bevorzugt. Flüssige Säuren oder in einem der nachstehend genannten inerten Lösungsmittel gelöste feste Säuren werden in äquimolaren Mengen zugesetzt. Bevorzugt werden wasserfreie Schwefelsäure, Essigsäure, Propionsäure, Phosphorsäure, Malonsäure und Benzoesäure, besonders bevorzugt wird wasserfreie Oxalsäure.

Als inerte Lösungsmittel eignen sich chlorierte Kohlenwasserstoffe wie Methylenchlorid, Chloroform, Tetrachlorkohlenstoffe, 1,1,1-Trichlorethan, Hexachlorethan, Tetrachlorethylen und Chlorbenzol; Ether wie Diethylether, Diisopropylether, Methyl-tert.-butylether, Tetrahydrofuran und Dioxan; Carbonsäureester wie Essigsäuremethylester und Essigsäureethylester; Kohlenwasserstoffe wie Toluol und Petrolether, bevorzugt werden Ether, beispielsweise Diethylether, Diisopropylether, Methyl-tert.-butylether, Tetrahydrofuran

und Dioxan, Chlorkohlenwasserstoffe wie Methylenchlorid und Chloroform oder Carbonsäurester, z.B. Essigsäureethylester.

Der Vorteil des erfindungsgemäßen Verfahrens gegenüber den bisher bekannten Methoden zur Herstellung von O-substituierten Hydroxylaminen I besteht in der breiten Anwendung der Synthese, insbesondere auch zur Herstellung von O-substituierten Hydroxylaminen der allgemeinen Formel I mit empfindlichen Resten R.

Die Spaltung der cyclischen Imidether II gelingt einfach und ohne Nebenreaktionen. Besonders einfach gestaltet sich die Abtrennung des Spaltproduktes von O-substituiertem Hydroxylamin I.

Das erfindungsgemäße Verfahren bietet die Möglichkeit, komplexe oder empfindliche O-substituierte Hydroxylamine I, wie beispielsweise Phenethoxyamine oder Phenoxyethoxyamine, die nach bisher bekannten Verfahren nicht erhalten werden konnten, in guten Ausbeuten herzustellen.

Insgesamt zeichnet sich das erfindungsgemäße Verfahren durch einfache Verfahrensschritte und bequeme Handhabung der Reaktionsmischungen aus, da keine voluminösen Niederschläge auftreten, so daß sich die Synthese einfach auch in großtechnischem Maßstab durchführen läßt.

Die O-substituierten Hydroxylamine I und IV und deren Salze sind wichtige Zwischenprodukte, insbesondere zur Herstellung von Pflanzenschutzmitteln (DE-A-31 21 355 und EP-A-01 42 741).

Beispiele

Die $^{1}H$-NMR-Spektren wurden, falls nichts anderes vermerkt ist, in Perdeutero-Dimethylsulfoxid ($D_6$-DMSO) als Lösungsmittel mit Tetramethylsilan als innerem Standard aufgenommen. Die chemischen Verschiebungen sind jeweils in δ[ppm] angegeben. Für die Signalstruktur wurden folgende Abkürzungen verwendet:
s = Singulett, d = Dublett, t = Triplett, q = Quartet und m = Multiplett

Herstellung der cyclischen Imidether II

2,3 mol cyclisches N-Hydroxyimid und 214 g Kaliumcarbonat in 2,3 l N-Methylpyrrolidon wird bei 40° C mit 2,3 mol Alkylchlorid (oder Alkylbromid) versetzt. Nach 4 stündigem Rühren bei 50° C wird die Mischung in Eiswasser gegossen, das Produkt abgesaugt und mit Wasser gewaschen.

Die Ergebnisse sind in Tabelle 1 zusammengestellt.

Tabelle 1

| Vor-produkt Nr. | R | 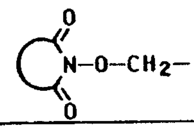 | Fp [°C] | $^1$H-NMR | Ausbeute [%] |
|---|---|---|---|---|---|
| II/1 | $-CH(OCH_2CH_3)_2$ | 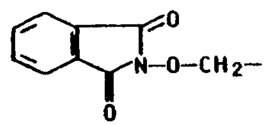 | | 1,10 (t), 1,10 (t) 3,48-3,74 (m) 4,15 (d), 4,86 (t) 7,82 (s) | 88 |
| II/2 | $o-F-C_6H_4-O-CH_2-$ | " | 119-121 | | 90 |
| II/3 | $o-Cl-C_6H_4-CH_2-$ | " | 73- 74 | | 78 |
| II/4 | 2-Tetrahydrofuryl | " | 98-100 | | 88 |
| II/5 | $-CH_2-O-$(2-Naphthyl) | " | > 230 | | 87 |
| II/6 | $m-CF_3-C_6H_4-O-CH_2-$ | " | 175-176 | | 82 |
| II/7 | Cyclopropyl | " | 78- 80 | | 86 |
| II/8 | $p-Cl-C_6H_4-CH_2-$ | " | | 3,06 (s), 4,36 (s), 7,37 (d), 7,83 (d), 7,87 (s) | 86 |
| II/9 | $-CH_2-CH_2-O-CH_2-CH_3$ | " | | 1,11 (t), 1,93 (t), 3,44 (q), 3,55 (t), 4,23 (t), 7,87 (s) | 81 |
| II/10 | 2,2-Dichlorcyclopropyl | " | | 1,53 (t), 1,84 (dd), 2,22-2,31 (m), 4,11 (dd), 4,50 (dd), 7,90 (s) | 87 |
| II/11 | $o-Cl-C_6H_4-O-CH_2-$ | " | 127-129 | | 99 |
| II/12 | $m-Cl-C_6H_4-O-CH_2-$ | " | | 4,33 (t), 4,53 (t) 6,84-7,03 (m), 7,29 (t) 7,87 (s) | 97 |
| II/13 | $-CH_2F$ | " | Öl | | 70 |

Tabelle 1 (Fortsetzung)

| Vorprodukt Nr. | R | 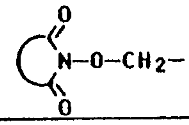 | Fp [°C] | $^1$H-NMR | Ausbeute [%] |
|---|---|---|---|---|---|
| II/14 | m-Cl-$C_6H_4$-S-$CH_2$- | 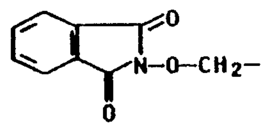 | 73- 75 | | 80 |
| II/15 | Cyclohexyl | ″ | 91- 93 | | 95 |
| II/16 | -$CH_2$-$CH_2$CN | ″ | 103-105 | | 89 |
| II/17 | -$CONH_2$ | ″ | 190-192 | | 81 |
| II/18 | -C≡C-$CH_3$ | ″ | Öl | | 72 |
| II/19 | 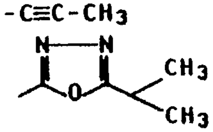 | ″ | 115-116 | | 97 |
| II/20 | 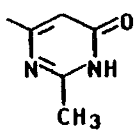 | ″ | 240-241 | | 85 |
| II/21 | 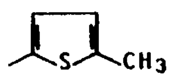 | ″ | Öl | | 75 |
| II/22 | trans-CH=CHCl | ″ | 132-134 | | 99 |
| II/23 | cis-CH=CHCl | ″ | | | 98 |
| II/24 | -COOC($CH_3$)$_3$ | ″ | 146-147 | | 96 |
| II/25 | p-F-$C_6H_4$- | ″ | 160-162 | | 99 |
| II/26 | p-$CH_3$-$C_6H_4$- | ″ | 144-146 | | 100 |
| II/27 | p-$NO_2$-$C_6H_4$- | ″ | 193-195 | | 99 |
| II/28 | p-CN-$C_6H_4$- | ″ | 197-199 | | 100 |
| II/29 | m-Cl-$C_6H_4$- | ″ | 138-140 | | 99 |

Tabelle 1 (Fortsetzung)

| Vorprodukt Nr. | R | 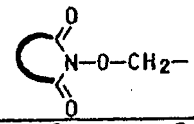 | Fp [°C] | $^1$H-NMR | Ausbeute [%] |
|---|---|---|---|---|---|
| II/30 | $-CH=CH_2$ | 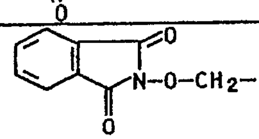 | 58- 61 | | 98 |
| II/31 | $-CH_2-OCH_3$ | " | 91- 93 | | 80 |
| II/32 | $-COOCH_2-CH_3$ | " | 85- 91 | | 98 |
| II/33 | $-CH_2-C_6H_5$ | " | | 3,05(t), 4,40(t), 7,20-7,38(m), 7,87(s) | 91 |
| II/34 | $-CH_2-CH=CH_2$ | " | | 2,43-2,53(m), 4,20(t), 5,08(dd), 5,21(dd), 5,81-5,99(m), 7,87(s) | 73 |
| II/35 | $-C\equiv CH$ | " | 150-151 | | 99 |
| II/36 | trans-$CH=CHCH_3$ | " | 109-113 | 1,65(d), 4,59(d), 5,66-5,85(m), 7,89(s) | 97 |
| II/37 | 5-Chlor-thien-2-yl | " | | 5,28(s), 7,05(d), 7,16(d), 7,85(s) | 98 |
| II/38 | $3,4-Cl_2-C_6H_3-$ | " | 181-183 | | 99 |
| II/39 | $4-CH_3O-C_6H_4-$ | " | 140-142 | | 98 |
| II/40 | $3-CF_3-C_6H_4-$ | " | 105-106 | | 98 |
| II/41 | $4-Cl-C_6H_4-$ | " | 136-137 | | 99 |
| II/42 | $N\equiv C-$ | " | 141-145 | | 82 |
| II/43 | Phenyl | 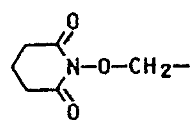 | 133-136 | | 87 |
| II/44 | Phenyl | 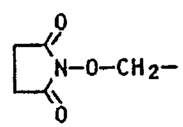 | 140-142 | | 97 |

Herstellung der O-substituierten Hydroxylamine I

Beispiel 1 bis 6

170 mmol Imidether II in 75 ml 2-Aminoethanol wird 2 Stunden bei 60 °C gehalten. Nach dem Eingießen der Reaktionsmischung in Wasser und Extrahieren des Produktes mit Methylenchlorid wird anschließend wie üblich aufgearbeitet.

Die Ergebnisse sind in Tabelle 2 zusammengestellt.

Tabelle 2

Verbindungen I $H_2N-O-CH_2-R$

| Beispiel Nr. Verbindung Nr. | Vorprodukt Nr. | R | $^1H$-NMR [ppm] | Ausbeute [%] |
|---|---|---|---|---|
| 1 | II/8 | 4-Chlorbenzyl | 2,85(t), 3,84(t), 5,37(s, breit), 7,11(d), 7,22(d) in $CDCl_3$ | 83 |
| 2 | II/4 | 2-Tetrahydrofuryl | 1,53-2,00(m), 3,60-4,00(m), 4,09-4,12(m), 5,57(s, breit) in $CDCl_3$, | 67 |
| 3 | II/33 | Benzyl | 2,80(t), 3,70(t), 5,98(s), 7,13-7,31(m), | 98 |
| 4 | II/37 | 5-Chlor-thien-2-yl | 4,71(s), 5,48(s, breit), 6,80(m) | 90 |
| 5 | II/19 | 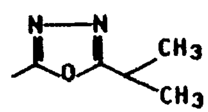 | 1,41 (d), 3,21 (m), 4,84 (s), 5,82 (s, breit) | 82 |
| 6 | II/19 | 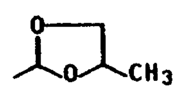 | 1,28(d), 1,32(d), 1,90-2,00(m), 3,32-3,42(m), 3,78-3,88(m), 4,09-4,27(m), 5,00-5,12(t), 5,48(s) | 85 |

Beispiel 7

Herstellung von Aminooxyessigsäure-tert.-butylester (Verbindung 7)

1,06 kg (3,8mol) N-Phthalimido-oxyessigsäure-tert.-butylester in 2 1 Diethylether wird mit 230 g (3,8 mol) 2-Aminoethanol versetzt. Die Mischung wird 4 Stunden unter Rückfluß erhitzt, das Spaltprodukt abgesaugt und der Rückstand destilliert (41 bis 42°C, 0,4 Torr). Die Ausbeute beträgt 80 %; $^1$H-NMR ($CDCl_3$): $\delta$ = 1,49(s), 4,13 (s), 5,85 (s, breit).

Beispiel 8

Herstellung von 2,2-Diethoxyethoxyamin (Verbindung 8)

Analog Beispiel 7 wird N-(Diethoxyethyloxy)-phthalimid umgesetzt und aufgearbeitet. Man erhält Verbindung 8 als Öl in 96 %iger Ausbeute. $^1$H-NMR ($CDCl_3$): $\delta$ = 1,22 (t), 1,22 (t), 3,53-3,64 (m), 3,73 (m), 4,74 (t), 5,60 (s).

Herstellung von Salzen der O-substituierten Hydroxylamine I

Beispiel 9 bis 49

Darstellung der Hydrochloride der Hydroxylamine I

Eine Lösung aus 2,2 mol eines Imidethers II und 1,5 Liter Essigsäureethylester wird mit 2,4 mol 2-Aminoethanol versetzt und 3 bis 4 Stunden bei 60°C gerührt. Danach wird das Gemisch auf 10°C abgekühlt, wobei das N-(Hydroxy-ethoxy)-phthalimid ausfällt und abgetrennt wird. In das Filtrat wird Chlorwasserstoff-Gas eingeleitet. Der entstandene Niederschlag des O-substituierten Hydroxylamin-hydrochlorids wird wie üblich aufgearbeitet.

Die Ergebnisse sind in Tabelle 3 zusammengestellt.

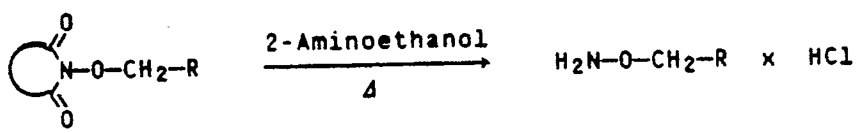

Tabelle 3

Salze der Verbindungen I $H_2N-O-CH_2-R \times (HCl)_q$

| Beispiel Nr. Verbindung Nr. | R | q | Fp. [°C] | $^1H$-NMR [ppm] | Ausbeute [%] |
|---|---|---|---|---|---|
| 9 | p-F-$C_6H_4$- | 1 | 232-234 | | 95 |
| 10 | o-F-$C_6H_4$-O-$CH_2$- | 1 | 170-172 | | 92 |
| 11 | p-$CH_3$-$C_6H_4$- | 1 | 211-213 | | 90 |
| 12 | p-$NO_2$-$C_6H_4$- | 1 | 202-204 | | 89 |
| 13 | p-CN-$C_6H_4$- | 1 | 192-195 | | 87 |
| 14 | m-Cl-$C_6H_4$- | 1 | 194-198 | | 94 |
| 15 | -$CH_2$-O-$CH_3$ | 1 | | 3,29(s), 3,57(t), 4,19(t), 11,21(s, breit) | 95 |
| 16 | Cyclopropyl | 1 | 150-151 | | 81 |
| 17 | -$CH_2$-O-(2-Naphthyl) | 1 | 200-202 | | 82 |
| 18 | m-$CF_3$-$C_6H_4$-O-$CH_2$- | 1 | 114-117 | | 83 |
| 19 | -$CH_2$-$CH_2$-$CH_2$-O-$CH_2$-$CH_3$ | 1 | 98-100 | | 84 |
| 20 | m-Cl-$C_6H_4$-S-$CH_2$- | 1 | 148-150 | | 90 |
| 21 | (Z)-CH=CHCl | 1 | 175-176 | | 98 |
| 22 | (E)-CH=CHCl | 1 | 177-179 | | 97 |
| 23 | 2,2-(Dichlorcyclopropyl) | 1 | | 1,60(t), 1,84(dd), 2,10-2,26(m), 4,05(dd), 4,28(dd), 11,27(s, breit) | 82 |
| 24 | -$CH_2$-F | 1 | 190-191 | 4,27(dd), 4,39(dd), 4,57(dd), 4,77(dd), 11,27(s, breit) | 91 |

Tabelle 3 (Fortsetzung)

| Beispiel Nr. Verbindung Nr. | R | q | Fp. [°C] | $^1$H-NMR [ppm] | Ausbeute [%] |
|---|---|---|---|---|---|
| 25 | o-Cl-$C_6H_4$-O-$CH_2$- | 1 | 172-174 | | 87 |
| 26 | m-Cl-$C_6H_4$-O-$CH_2$- | 1 | | 4,30(m), 4,44(m), 6,93-7,07(m), 7,33(t), 11,30(s, breit) | 88 |
| 27 | -$CH_2$-CH=$CH_2$ | 1 | 141-143 | | 76 |
| 28 | trans-CH=CH-$CH_3$ | 1 | 158-160 | 1,71(d), 4,48(d), 5,50-5,64(m), 5,83-6,00(m), 11,09(s, breit) | 93 |
| 29 | m-$CF_3$-$C_6H_4$- | 1 | 163-165 | | 95 |
| 30 | p-Cl-$C_6H_4$- | 1 | 219-220 | | 98 |
| 31 | p-Methoxy-$C_6H_4$- | 1 | 190-191 | | 90 |
| 32 | 3,4-Dichlor-$C_6H_3$- | 1 | 183-185 | | 89 |
| 33 | 2,6-Dichlor-$C_6H_3$- | 1 | 196-199 | | 91 |
| 34 | -Cyclohexyl | 1 | 174 | | 89 |
| 35 | -CN | 1 | 114-116 | | 85 |
| 36 | -$CH_2$-$CH_2$-CN | 1 | 138-140 | | 89 |
| 37 | -$CONH_2$ | 1 | 132-135 | | 81 |
| 38 | Pyrid-3-yl | 2 | 187-188 | | 70 |
| 39 | -$CH_2CH_3$ | 1 | 164-165 | | 85 |
| 40 | 2-Phenoxyethyl | 1 | 119-120 | | 87 |
| 41 | -C≡CH | 1 | 163-164 | | 79 |
| 42 | (4-Phenoxy-phenoxy)-methyl | 1 | 183-184 | | 91 |

| Beispiel Nr. Verbindung Nr. | R | q | Fp. [°C] | $^1$H-NMR [ppm] | Ausbeute [%] |
|---|---|---|---|---|---|
| 43 | $-CH(CH_3)_2$ | 1 | 126-128 | | 82 |
| 44 | $m-F-C_6H_4-$ | 1 | 204-206 | | 98 |
| 45 | $o-F-C_6H_4-$ | 1 | 177-178 | | 97 |
| 46 | 3-Phenoxy-n-propyl | 1 | 177-178 | | 95 |
| 47 | $m-NO_2-C_6H_4$ | 1 | 172-175 | | 85 |
| 48 | $H_2N-O-CH_2-$ | 2 | 218-220 | | 93 |
| 49 | $H_2N-O-CH_2-CH=CH-$ | 2 | 176-180 | | 91 |

Herstellung der Oxalate der Hydroxylamine I

Beispiel 50

18,4 g (60 mmol) o-Chlorphenethoxyphthalimid wird 4 Stunden bei 50° C in 27 ml 2-Aminoethanol gerührt. Nach Verdünnung mit 200 ml Essigsäureethylester wird dreimal mit je 100 ml Wasser gewaschen und über Natriumsulfat getrocknet. Dem Filtrat wird unter Eiskühlung 11,3 g (125,5 mmol) Oxalsäure gelöst in 100 ml Essigsäureethylester zugetropft und 10 Minuten bei Raumtemperatur gerührt, das Produkt abgesaugt und mit Essigsäureethylester gewaschen. Die Ausbeute an Verbindung 50 beträgt 89 %; Fp. 120 bis 122° C.

Beispiel 51

Analog Beispiel 50 wird aus p-Chlorbenzyloxyphthalimid das Oxalat hergestellt. Das O-p-Chlorbenzylhydroxylamin-oxalat (Verbindung 51) fällt in 98 %iger Ausbeute an; Fp.: 143° C.

Analog Beispiel 50 und 51 sind die Verbindungen 52 und 53 hergestellt worden. Die Ergebnisse sind in Tabelle 4 zusammengestellt.

**Tabelle 4**

$H_2N-O-CH_2-R \times (COOH)_2$

| Beispiel Nr. Verbindung Nr. | R | Fp. [°C] | Ausbeute [%] |
|---|---|---|---|
| 52 | 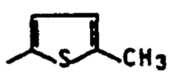 | 106-111 | 67 |
| 53 | $CH_3-C\equiv C-$ | 114-117 | 72 |

**Ansprüche**

1. Verfahren zur Herstellung von O-substituierten Hydroxylaminen der allgemeinen Formel I,

$$H_2N-O-CH_2-R \qquad (I)$$

in der R Wasserstoff oder einen organischen Rest bedeutet, und deren anorganischen und organischen Salzen, durch Umsetzung eines cyclischen Imidethers II

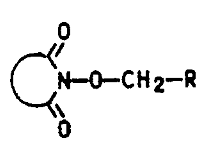 (II)

einer 1,4- oder 1,5-Dicarbonsäure, der eine Ethergruppierung $-O-CH_2-R$ enthält, mit einer basischen

Verbindung, dadurch gekennzeichnet , daß man als basische Verbindung einen primären aliphatischen Aminoalkohol III,

$$H_2N\text{-}A\text{-}OH \qquad (III)$$

in der -A- einen zweiwertigen aliphatischen Rest bedeutet, verwendet und die Verbindungen I danach gegebenenfalls in ihre Salze überführt.

2. Verfahren nach Anspruch 1, dadurch gekennzeichnet , daß man die Umsetzung im Falle wasserunlöslicher Verbindungen I unter Verwendung eines molaren Überschusses des Aminoalkohols III vornimmt.

3. Verfahren nach Anspruch 2, dadurch gekennzeichnet , daß man das hiernach erhaltene Reaktionsgemisch zur Entfernung wasserlöslicher Anteile mit Wasser extrahiert.

4. Verfahren nach Anspruch 1, dadurch gekennzeichnet , daß man die Umsetzung von äquimolaren Mengen des Aminoalkohols III und des Imidethers II in Gegenwart eines inerten Lösungsmittels vornimmt und danach durch Zugabe einer Säure die O-substituierten Hydroxylamine I als Salz ausfällt.

5. Verfahren nach den Ansprüchen 1 bis 4, dadurch Gekennzeichnet, da8 man als primären aliphatischen Aminoalkohol III eine Verbindung der Formel III verwendet

$$H_2N\text{-}A\text{-}OH \qquad (III)$$

in der -A- für $-(CH_2)_n-$, $-CH_2CH(CH_3)CH_2-$ oder $-(CH_2CH_2O)_m-CH_2CH_2-$steht, wobei m = 1 oder 2 und n = 2 bis 4 bedeuten.

## Claims

1. A process for the preparation of an O-substituted hydroxylamine of the formula I

$$H_2N\text{-}O\text{-}CH_2\text{-}R \qquad (I)$$

where R is hydrogen or an organic radical, and its inorganic and organic salts, by reacting a cyclic imidoether II

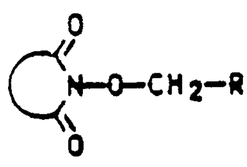

(II)

of a 1,4- or l,5-dicarboxylic acid, the said imidoether containing an ether group $-O-CH_2-R$, with a basic compound, wherein a primary aliphatic aminoalcohol III

$$H_2N\text{-}A\text{-}OH \qquad (III)$$

where -A- is a divalent aliphatic radical, is used as the basic compound, and the compound I is, if required, then converted to its salts.

2. A process as claimed in claim 1, wherein, in the case of water-insoluble compounds I, the reaction is carried out using a molar excess of the aminoalcohol III.

3. A process as claimed in claim 2, wherein the resulting reaction mixture is extracted with water in order to remove water-soluble components.

4. A process as claimed in claim 1, wherein the reaction of equimolar amounts of the aminoalcohol III and of the imidoether II is carried out in the presence of an inert solvent, and the O-substituted hydroxylamine I is then precipitated as a salt by adding an acid.

5. A process as claimed in claim 1 or 2 or 3 or 4, wherein the primary aliphatic aminoalcohol III used is a compound of the formula III

$$H_2N-A-OH \qquad (III)$$

where -A- is $-(CH_2)_{n}-$, $-CH_2CH(CH_3)CH_2-$ or $-(CH_2CH_2O)_m -CH_2CH_2-$, m is 1 or 2 and n is 2, 3 or 4.

## Revendications

1. procédé de préparaticn d'hvdroxvlamines O-substituées de formule générale I

$$H_2N-O-CH_2-R \qquad (I)$$

dans laquelle A représente un atome d'hydrogène ou un reste organique, et de leurs sels inorganiques et organiques par réaction, avec un composé basique, d'un imido-éther cyclique II

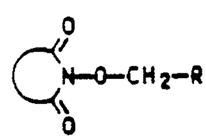

(II)

d'un acide 1,4- ou 1-5-dicarboxylique, qui contient un groupement éther $-O-CH_2-R$, caractérisé en ce qu'on utilise, comme composé basique, un amino-alcool aliphatique primaire III

$$H_2N-A-OH \qquad (III)$$

où -A- représente un reste aliphatique bivalent, après quoi on transforme éventuellement les composés I en leurs sels.

2. Procédé selon la revendication 1, caractérisé en ce qu'en cas de composés I insolubles dans l'eau, on procède à la réaction en utilisant un excès molaire de l'amino-alcool III.

3. Procédé selon la revendication 2, caractérisé en ce qu'on extrait à l'eau le mélange réactionnel ainsi obtenu pour en éliminer les parties solubles dans l'eau.

4. Procédé selon la revendication 1, caractérisé en ce qu'on procède à la réaction de quantités équimolaires de l'amino-alcool III et de l'imido-éther II en présence d'un solvant inerte, puis on précipite les hydroylamines O-substituées sous forme de sel par addition d'un acide.

5. Procédé selon l'une des revendications 1 à 4, caractérisé en ce qu'on utilise, comme amino-alcool aliphatique primaire III, un composé de formule III

$$H_2N-A-OH \qquad (III)$$

dans laquelle -A- représente $-(CH_2)_n-$, $-CH_2CH(CH_3)CH_2-$ou $-(CH_2\ CH_2O)_m-CH_2CH_2-$, m étant mis pour 1 ou 2 et n pour 2 à 4.